**Europäisches Patentamt**

**European Patent Office**

**Office européen des brevets**

(11) Publication number: **0 195 455**

**B1**

(12) **EUROPEAN PATENT SPECIFICATION**

(45) Date of publication of patent specification: **28.02.90**　　(51) Int. Cl.$^5$: **A 61 B 5/03**

(21) Application number: **86103872.7**

(22) Date of filing: **21.03.86**

(54) **Cranial screw.**

(30) Priority: **22.03.85 US 714669**

(43) Date of publication of application:
**24.09.86 Bulletin 86/39**

(45) Publication of the grant of the patent:
**28.02.90 Bulletin 90/09**

(84) Designated Contracting States:
**CH DE FR GB IT LI NL**

(56) References cited:
**WO-A-83/03190
DE-A-2 621 909
FR-A-2 328 876
FR-A-2 389 362
US-A-4 068 555
US-A-4 206 762**

(73) Proprietor: **CODMAN & SHURTLEFF INC.
Pacella Park Drive
Randolph Massachusetts 02368 (US)**

(72) Inventor: **Kraus, Robert George
46 Doral Lane
Attleboro Massachusetts 02703 (US)**

(74) Representative: **Strehl, Schübel-Hopf, Groening
Maximilianstrasse 54 Postfach 22 14 55
D-8000 München 22 (DE)**

Courier Press, Leamington Spa, England.

**Description**

The present invention relates to the cranial screw having a hollow shank which has a distal threaded portion for screwing into a hole in the cranium, and a first drive head, a second drive head separated by a frangible portion from said first drive head, which frangible portion breaks at a predetermined maximal torque.

FR—A 2 328 876 discloses a security plug used in concrete structures providing a screw with a second drive head separated by a frangible portion from a first screw head, which frangible portion breaks at a predetermined maximal torque. This second drive head and the frangible portion provide the advantage that, if excess torque is placed on said screw during insertion into the hole of a concrete wall, said frangible portion will break leaving said first drive head and said threaded portion intact so that the remainder of said broken screw can be removed using said first drive head.

Cranial screws are inserted into a surgical bore through the cranium to provide access to the interior of the cranium so that a variety of procedures can be performed, for example, so that the internal cranial pressure may be monitored by equipment external to the cranium. Traditional cranial screws are made of stainless steel and have a hollow shank, which has a distal portion for insertion into a hole in the cranium, and a drive head. The distal portion may have a self-tapping thread which is tapped into the cranium as the head of the screw is turned with a wrench. Stainless steel is very strong and is made to withstand even very large torquing forces that must be applied to insert the self-tapping thread into the cranium.

However, stainless steel cranial screws interfere with an X-ray or a magnetic field, thus, making diagnosis of the patient with the emerging technologies of CT and MR scanning difficult, if not impossible. CT scanning is essentially a computer-assisted X-ray machine which is used to diagnose conditions of the body. An MR scanner uses a resonant magnetic field to diagnose conditions of the body.

Because of the increasing importance of CT and MR scanning, an effort has been made to make surgical appliances which are traditionally applied to the cranium out of materials which are compatible with CT and MR scanning, that is, materials which are transparent to X-ray and magnetic fields. A variety of plastics have been suggested for this purpose. However, plastics do not have the strength of stainless steel. It is possible that if a traditional stainless steel screw is fabricated in plastic, the forces experienced during insertion could be large enough to shear the threaded portion of the screw. This is an undesirable result, because it is very difficult to remove the remaining threaded portion of a screw that is left in the cranium.

It would be desirable to have a cranial screw that was CT and MR compatible but at the same time could be easily removed if the screw were overtightened.

Throughout this patent application the word "distal" will be used to indicate that portion of the screw which faces closer to the cranium into which the screw is inserted, and "proximal" will be used to indicate that portion of the screw which faces away from the cranium.

Summary of the Invention

The present invention provides a cranial screw which is CT and MR compatible and which is specially designed with double drive heads separated axially by a frangible portion of the shank. Thus, if the screw breaks, it will break at a predetermined portion so that an emergency removal drival head is left attached to the broken shank and so that the broken portion of the screw may be easily removed from the cranium.

The screw preferably includes a hollow plastic shank which has a lumen for providing access to the interior of the cranium. The shank has a distal end adapted for insertion into the cranium and a proximal end extending outside the cranium. The distal end of the screw has a threaded portion. An emergency removal drive head is integrally connected about the exterior of the shank adjacent the threaded portion. An insertion drive head is integrally connected about the exterior of the shank and spaced proximally from the removal drive head. A frangible portion of the shank integrally connects the emergency removal drive head and the insertion drive head. If excessive torque is placed on the screw during insertion, the shank will break at a preferential position on the frangible portion of the shank, leaving the emergency removal drive head and the threaded distal portion of the shank in the cranium, so that the emergency removal drive head can be used to remove the broken portion of the shank from the cranium. The wall strength of the frangible portion is less than that of the threaded distal end of the shank. The screw is preferably made of one uniform material. However, the frangible section may be made of a different and frangible material. The screw is made of a material or materials which are transparent to X-ray or magnetic fields.

The threads are preferably self tapping. The distal tip of the shank is preferably chamfered for easy insertion. The lumen of the hollow shank is preferably slightly tapered to facilitate molding.

The proximal end of the shank preferably extends proximally from the insertion drive head, so that it may be connected to equipment external to the cranium. This external portion may be configured to accept a standard luer lock connection.

The distal end of the emergency removal drive head may be formed into a circular flange, and a washer may be placed about the distal end of the shank to provide a tight seal between the flange and the cranium when the screw is tightened into place.

It cane be seen that the present invention provides a cranial screw which is compatible with CT and MR scanning and which is specially designed to accommodate the weaker materials, such as plastics, from which it is made. The special design will cause the screw to break if it is over-torqued in a part of the screw which leaves sufficient structure on the portion of the screw that is sheared off in the cranium, so that it may be easily removed with a socket driver.

Brief Description of the Drawings

These and other advantages and features of the present invention will become apparent from the following description of certain embodiments of the invention taken in conjunction with the following drawings in which:

Figure 1 shows a perspective view of the cranial screw of the present invention;

Figure 2 shows perspective views of the cranial screw of the present invention inserted into the cranium; and,

Figure 3 shows a cross-sectional view of the cranial screw of the present invention.

Detailed Description of the Preferred Embodiment

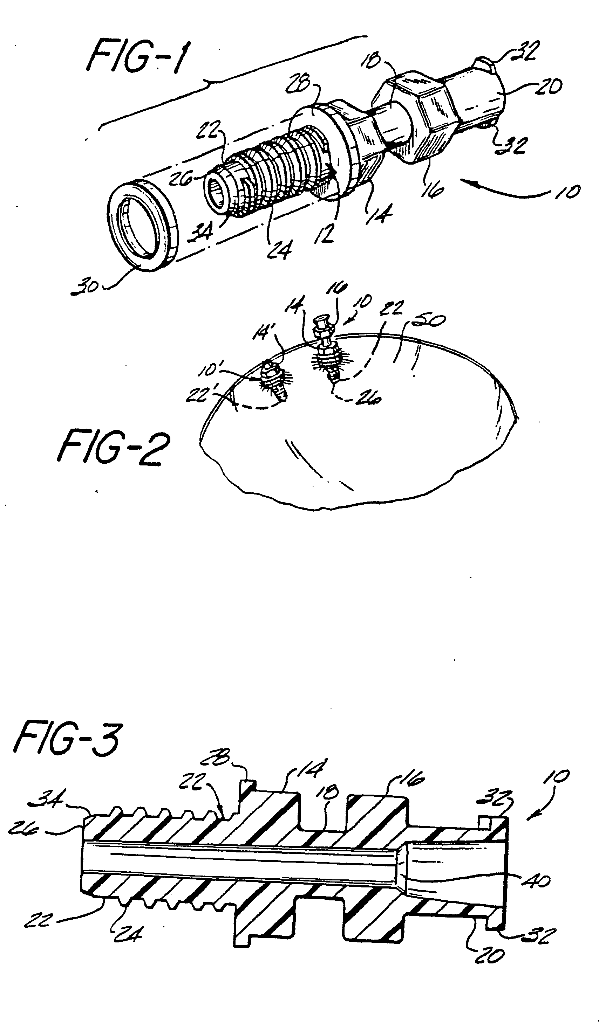

Referring now to Figure 1, there is shown the cranial screw 10 of the present invention having a hollow shank 12 and emergency removal drive head 14 and insertion drive head 16 separated by a frangible portion 18 of shank 12. Shank 12 has a proximal portion 20 extending proximally from insertion drive head 16.

The distal portion 22 of shank 12 includes threads 24 which are preferably self-tapping threads. Threads 24 extend from the distal tip 26 of screw 10 up to the distally-facing surface of emergency removal drive head 14. The distally-facing surface of emergency removal drive head 14 may be formed into a circular flange 28. Washer 30 is placed over distal portion 22 to provide a tight seal between flange 28 and the surface of the cranium when screw 10 is tightened down against the cranium. This permits a tight fluid connection through the cranium without leaks around the outside of the screw so that, for example, accurate measurements of intracranial pressure (ICP) can be made.

Emergency removal drive head 14 and insertion drive head 16 are preferably formed into a hexagonal cross-section so that they may be easily received within a standard socket driver.

Proximal portion 20 extends proximally from insertion drive head 16 and is adapted for connection to other equipment external to the cranium. Preferably, proximal portion 20 is formed as a standard luer lock with luer lock tabs 32 extending radially from the proximal tip of proximal portion 20.

The distal tip 26 of cranial screw 10 preferably has a chamfer 34 for easy insertion into a hole in the cranium.

Referring now to Figure 3, there is shown a cross-sectional view of the cranial screw of the present invention.

It can be seen that the thickness of the wall of the screw shank, including the thickness added by threads 24, is thicker in the distal portion 22 than in frangible portion 18. In the preferred embodiment, the wall in distal portion 22 is 5% to 40% stronger in torsion than the wall in frangible portion 18. It is believed that this difference in wall thickness is not critical, but that any appropriate thicknesses could be used that would permit the entire shank to be strong enough to withstand the torque applied during normal insertion of the cranial screw into the cranium and at the same time would permit the shank of the screw to break preferentially in frangible portion 18 if it were to be over-torqued. Alternatively, a groove cut may be formed on the outside wall of frangible portion 18 to provide a predetermined weak point. Other frangible configurations could also be used.

It will be noted from Figure 3 that the internal diameter of the shank tapers from the distal portion 22 toward the proximal portion 20 to facilitate molding of cranial screw 10. This tapering permits the screw to be easily removed from the mold.

It will also be noted from figure 3 that the internal diameter of the screw in proximal portion 20 is greater than the internal diameter in the remaining portion of the shank, and that the transition zone 40 appears just proximally of insertion drive head 16. This transition zone is included so that the dimensions of proximal portion 20 will be those of a standard luer fitting. Thus, the cranial screw can be easily adapted to standard external equipment without having to compromise the frangible design of the screw.

The screw is preferably made of one uniform material such as polyether-imide. I prefer the brand of polyether-imide sold under the trademark "ULTEM" by the General Electric Company. Other materials from which the screw can be made are polycarbonate, polyamide-imide, or other high strength engineering resins.

Referring now to Figure 2, the operation of the present cranial screw 10 will be explained. Figure 2 shows a cranium 50 with a complete cranial screw 10 inserted through a hole in the cranium which is made by standard surgical techniques well known to those skilled in this art. Distal portion 22 of cranial screw 10 is shown in dashed lines inserted into the cranium. Distal tip 26 of screw 10 may extend all the way through the cranium and project into the cranial space or it may stop inside the cranial bore and be flush with the inside surface of the cranium, as desired. Another cranial screw 10' which has sheared off during insertion is shown in cranium 50. It can be seen that cranial screw 10' has sheared off in the frangible portion between emergency removal drive head 14 and insertion drive head 16. It can be seen that emergency drive head has been left integrally connected to distal portion 22', so that the portion of cranial screw 10' that has sheared off may be easily removed by placing a socket wrench over emergency removal drive head 14' and merely unscrewing the portion of cranial screw 10' that has been left in the cranium.

Thus, the present invention provides a cranial screw which is made of materials which are compatible with CT and MR scanning. The structure of the cranial screw of the present invention is specially designed to recognize that CT and MR compatible materials may be weaker than the stainless steel that is used for traditional cranial screws, so that the cranial screw 10 of the present invention will break, if it is over-torqued during insertion, at a preferred position on the frangible portion 18 of shank 12, so that the portion which has sheared off may be easily removed.

While the present invention has been described in connection with certain preferred embodiments, those skilled in this art will appreciate that certain modifications may be made without departing from the scope of the present invention. It is, therefore, not intended that the present invention be limited except as set forth in the following claims.

## Claims

1. A cranial screw (10) having a hollow shank (12) which has a distal threaded portion (22) for screwing into a hole in the cranium (50), and a first drive head (16), characterized in that said screw (10) has a second drive head (14) separated by a frangible portion (18) from said first drive head (16), which frangible portion (18) breaks at a predetermined maximal torque, and said screw (10) is made of a material which will not show up on an X-ray or a magnetic resonance scan of the cranium (50).

2. The screw of Claim 1 wherein said frangible portion (18) is made of a different material from the remainder of said screw (10).

3. The screw of Claim 1 wherein said threaded portion (22) includes a self-tapping thread (24).

4. The screw of Claim 1 wherein said distal tip (26) is chamfered for easy insertion.

5. The screw of Claim 1 wherein said lumen is slightly tapered to facilitate molding.

6. The screw of Claim 1 wherein said proximal portion (20) extends proximally from said insertion drive head (16) and is adapted for connection to equipment external to the cranium (50).

7. The screw of Claim 6 wherein said extending proximal portion (20) end includes a standard luer lock connection (32).

8. The screw of Claim 1 wherein the distal portion (22) of said emergency removal drive head (14) is formed into a circular flange (28); and,

further including washer (30) to provide a tight seal between said flange (28) and the cranium (50) when said screw (10) is tightened.

9. The screw of Claim 1 wherein said distal portion (22) includes a first wall thickness and said frangible portion (18) includes a second wall thickness less than said first wall thickness.

## Patentansprüche

1. Schädelschraube (10) mit einem hohlen Schaft (12), die einen vorderen, mit Schraubgewinde versehenen Teil (22) zum Einschrauben in ein Loch in der Schädeldecke (50) sowie einen ersten Schraubkopf (16) aufweist, dadurch gekennzeichnet, daß die Schraube (10) einen zweiten Schraubkopf (14), hat, der durch einen zerbrechlichen Teil (18) von dem ersten Schraubkopf (16) getrennt ist, wobei der zerbrechliche Teil (18) bei einem vorbestimmten maximalen Drehmoment bricht und die Schraube (10) aus einen Werkstoff hergestellt ist, der bei einer Untersuchung der Schädeldecke (50) mittels Röntgenstrahlen oder kernmagnetischer Resonanz nicht dargestellt wird.

2. Schraube nach Anspruch 1, bei der der zerbrechliche Teil (18) aus einem gegenüber dem Rest der Schraube (10) unterschiedlichen Material hergestellt ist.

3. Schraube nach Anspruch 1, bei der der mit dem Schraubgewinde versehene Teil (22) ein Schneidgewinde (24) umfaßt.

4. Schraube nach Anspruch 1, bei der die vordere Spitze (26) zur Erleichterung des Einsetzens abgeschrägt ist.

5. Schraube nach Anspruch 1, bei der der genannte Hohlraum zur Erleichterung des Gießens geringfügig verjüngt ist.

6. Schraube nach Anspruch 1, bei der das hintere Ende (20) sich von dem Einsetzschraubkopf (16) nach hinten erstreckt und mit einer Ausrüstung außerhalb der Schädeldecke (50) verbindbar ist.

7. Schraube nach Anspruch 6, bei der der sich nach hinten erstreckende Teil (20) einen üblich Luer-Verriegelungsanschluß (32) umfaßt.

8. Schraube nach Anspruch 1, bei der das Vorderende (22) des im Notfall entfernbaren Schraubkopfes (14) als Ringflansch (28) ausgebildet ist und eine Unterlegscheibe (30) umfaßt, um eine feste Abdichtung zwischen dem Flansch (28) und der Schädeldecke (50) zu ermöglichen, wenn die Schraube (10) festgezogen wird.

9. Schraube nach Anspruch 1, bei der das Vorderende (22) eine erste Wandstärke umfaßt und der zerbrechliche Teil (18) eine zweite Wandstärke umfaßt, die geringer als die erste Wandstärke ist.

## Revendications

1. Vis crânienne (10) comportant une tige creuse (12), qui possède une partie distale filetée (22) destinée à être vissée dans un trou ménagé dans le crâne (50), et une première tête d'entraînement (16), caractérisée en ce que ladite vis (10) possède une seconde tête d'entraînement (14) séparée de ladite première tête d'entraînement (16) par une partie pouvant être rompue (18), qui se rompt lorsqu' apparaît un couple maximal prédéterminé, et que ladite vis (10) est réalisée en un matériau qui n'apparaît pas sur une radiographie ou lors d'une exploration par balayage à résonance magnétique du crâne (50).

2. Vis selon la revendication 1, dans laquelle ladite partie pouvant être rompue (18) est réalisée en un matériau différent du reste de ladite vis (10).

3. Vis selon la revendication 1, dans laquelle ladite partie filetée (22) comprend un filetage autotaraudeur (24).

4. Vis selon la revendication 1, dans laquelle ladite pointe distale (26) est biseautée de manière à faciliter l'insertion.

5. Vis selon la revendication 1, dans laquelle la cavité possède une légère conicité de manière à faciliter le moulage.

6. Vis selon la revendication 1, dans laquelle ladite partie proximale (20) se prolonge sur le côté proximal à partir de ladite tête d'entraînement pour l'insertion (16) et est adaptée pour être raccordée à un équipement situé à l'extérieur de crâne (50).

7. Vis selon la revendication 6, dans laquelle ladite extrémité de la partie proximale prolongée (20) comporte un élément de raccordement à verrouillage standard Luer (32).

8. Vis selon la revendication 1, dans laquelle la partie distale (22) de ladite tête d'entraînement pour un enlèvement d'urgence (14) est mise en forme de bride circulaire (28); et

il est prévu en outre une rondelle (30) destinée à établir une liaison étanche entre ladite bride (28) et le crâne (50) lors du serrage de ladite vis (10).

9. Vis selon la revendication 1, dans laquelle ladite partie distale (22) comporte une première épaisseur de paroi et ladite partie pouvant être rompue (18) possède une seconde épaisseur de paroi inférieure à ladite première épaisseur de paroi.

FIG-1

FIG-2

FIG-3